# EUROPEAN PATENT SPECIFICATION

(11) **EP 2 313 390 B1**
(45) Date of publication and mention of the grant of the patent: **19.09.2012**
(21) Application number: 09781555.9
(22) Date of filing: 06.08.2009
(51) Int. Cl.: C07D 401/04, A61K 39/385, A61P 25/34

(54) **PROCESS FOR THE PREPARATION OF NICOTINE-BASED HAPTENS**
VERFAHREN ZUR HERSTELLUNG VON HAPTENEN AUF NICOTINBASIS
PROCÉDÉ DE PRÉPARATION D'HAPTÈNES À BASE DE NICOTINE

(30) Priority: 08.08.2008 EP 08162095
(43) Date of publication of application: 27.04.2011
(73) Proprietor: Novartis AG, 4056 Basel (CH)
(72) Inventor: MARTIN, Benjamin, FR-68220 Leymen (FR)
(74) Representative: Finnie, Nicholas James
(86) International application number: PCT/EP2009/060203
(87) International publication number: WO 2010/015675

(56) References cited:
- US-A1- 2004 059 094
- US-A1- 2006 111 271
- LANGONE J J ET AL: "RADIOIMMUNOASSAY OF NICOTINE, COTININE, AND GAMMA-(3-PYRIDYL)-GAMMA-O XO-N-METHYLBUTYRAMIDE" METHODS IN ENZYMOLOGY, ACADEMIC PRESS INC, SAN DIEGO, CA, US, vol. 84, 1 January 1982 (1982-01-01), pages 628-640, XP009081629 ISSN: 0076-6879 cited in the application
- JONES, STEPHEN R. ET AL: "Improved iodine-125 radioimmunoassay for cotinine" CLINICAL CHEMISTRY (WASHINGTON, DC, UNITED STATES) , 31(6), 1076-7 CODEN: CLCHAU; ISSN: 0009-9147, 1985, XP002508991

## Description

### Field of the invention

This invention relates to a process for preparing nicotine-based haptens that are useful components of smoking cessation vaccines.

### Background to the invention

Tobacco is the world's most widely used addictive drug. The principal addictive component of tobacco is nicotine, an alkaloid derived from tobacco leaves. The smoking of tobacco in cigarettes is the single leading cause of preventable death in the United States and many other countries. While most smokers are aware that lung cancer, coronary heart disease, chronic lung disease and stroke can be caused by smoking, the majority of cigarette smokers who try to quit fail to do so.

Various behavioural and pharmacologic treatments are available to help smokers quit including nicotine replacement therapy (e.g. using nicotine gums or transdermal patches) and antidepressant treatment (e.g. using bupropion). However results are decidedly mixed. They can give rise to undesirable side effects. And they rely heavily on smokers maintaining their will power not to smoke.

US patent 6,932,971 discloses a therapeutic vaccine for smoking cessation. When administered the vaccine induces the production of the high levels of nicotine-specific antibodies that bind nicotine in the blood. As the complex of nicotine attached to an antibody is too large to pass the blood-brain-barrier, nicotine uptake into the brain and the subsequent stimulation of nicotine-perceptive neurons in the brain is believed to be significantly reduced or even prevented. In this way the addiction-driving and satisfaction-inducing stimulus of nicotine is minimized.

The vaccine of US 6,932,971 consists of a nicotine-based hapten linked via a chemical bridge to the surface of a phagus Qβ recombinant virus-like-particle produced in *E*. *coli.* The nicotine-based hapten is a known nicotine derivative, 1-(trans-3'-hydroxymethyl-nicotinyl)-6-hydroxy-succinimidyl-succinate (C₁₉H₂₃N₃O₆). This hapten is synthesized by reacting trans-3'-hydroxymethylnicotine with succinic anhydride to yield the succinylated hydroxymethyl-nicotine, O-succinyl-3'-hydroxymethyl-nicotine, based on the procedure disclosed in Langone et al "Radioimunno-assay of nicotine, cotine and γ-(3-pyridyl)-γ-oxo-N-methylbutyramide", Methods Enzymol. 84, pages 628-640, Academic Press 1982. This compound is then mixed with 1-ethyl-3-(3-dimethylaminopropyl)-carbodiimide (EDC) and N-hydroxysuccinimide (NHS) to give the N-hydroxysuccinimide ester of O-succinyl-3'-hydroxymethyl-nicotine, namely the aforementioned 1-(trans-3'-hydroxymethyl-nicotinyl)-6-hydroxysuccinimidyl-succinate.

The process disclosed in US 6,932,971 is undesirable for industrial production as the hapten that is prepared by the process can contain residual amounts of EDC, which is toxic and mutagenic. The hapten is also an inherently unstable molecule. This makes handling and preparation of the hapten difficult. There is therefore a need for a process for preparing the hapten that avoids or at least minimizes these problems.

### Summary of the Invention

In a first aspect, the present invention relates to a process for preparing a nicotine-based hapten of formula I 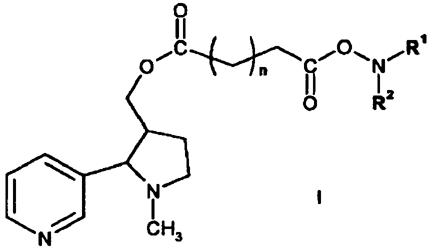 or a salt or solvate thereof

wherein n is an integer from 0 to 5, and R¹ and R² together form a N-bonded 5- to 10-membered heterocyclic group containing from 1 to 4 ring nitrogen atoms and optionally containing from 1 to 4 other heteroatoms selected from the group consisting of oxygen and sulfur, said heterocyclic group being optionally substituted at 1, 2 , 3 or 4 positions by halo, cyano, hydroxy, oxo, amino, aminocarbonyl, nitro, C₁-C₄-alkyl, C₁-C₄ alkoxy or C₃-C₆-cycloalkyl; the process comprising the steps of:

(a) reacting a compound of formula II 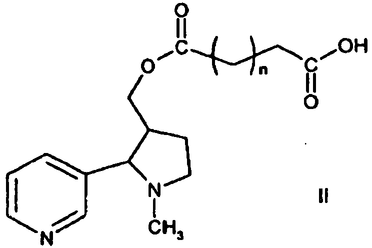 or a salt or solvate thereof, wherein n is an integer from 0 to 5,

with a compound of formula III 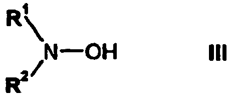 or a salt thereof,

wherein R¹ and R² are as hereinbefore defined,

and a polymer-supported coupling agent; and

(b) filtering the product of step (a) to give a compound of formula I in free, salt or solvate form, as hereinbefore defined.

In a second aspect the present invention relates to a nicotine-based hapten of formula I as hereinbefore defined obtainable or obtained by the aforementioned process for preparing a nicotine-based hapten of formula I. Preferably, the hapten is provided as a composition wherein the hapten is greater than 80, 85 or 90% pure, as determined, for example, by ¹H NMR.

In a third aspect the present invention provides a method for preparing a hapten-carrier conjugate, the method comprising covalently coupling a nicotine-based hapten of formula I obtained or obtainable by the process of the first aspect of the invention to one or more coat proteins of a virus like particle (VLP). In one embodiment, the VLP comprises coat proteins of an RNA phage, preferably phage Qβ.

### Detailed description of the invention

Terms used in the specification have the following meanings:

"Optionally substituted" as used herein means the group referred to can be substituted at one or more positions by any one or any combination of the radicals listed thereafter.

"Halo" or "halogen" as used herein may be fluorine, chlorine, bromine or iodine. Halo is suitably chlorine.

"C₁-C₅-alkyl" as used herein denotes straight chain or branched alkyl having 1 to 5 carbon atoms. C₁-C₅-alkyl is suitably methyl or ethyl.

"C₃-C₈-cycloalkyl" as used herein denotes cycloalkyl having 3 to 8 ring carbon atoms, for example a monocyclic group such as a cyclopropyl, cyclobutyl, cyclopentyl, cyclohexyl, cycloheptyl or cyclooctyl, or a bicyclic group such as bicycloheptyl or bicyclooctyl. C₃-C₈-cycloalkyl" is suitably C₅-C₆-cycloalkyl, especially cyclohexyl.

"N-bonded 5- to 10-membered heterocyclic group containing from 1 to 4 ring nitrogen atoms and optionally containing from 1 to 4 other heteroatoms selected from the group consisting of oxygen and sulfur" as used herein is a heterocyclic group that contains 5, 6, 7, 8 9, or 10 ring atoms, one of which is nitrogen and is attached to the oxygen atom of the ester group that is distal to the nicotinyl moiety of the compound of formula I, wherein optionally 1, 2 or 3 of the other ring atoms are nitrogen atoms and optionally 1, 2, 3 or 4 of the other ring atoms can be selected from oxygen and sulfur. The N-bonded 5- to 10-membered heterocyclic group may be, for example a saturated or an unsaturated, mono-cyclic or bicyclic heterocyclic group. The N-bonded 5- to 10-membered heterocyclic group is suitably a N-bonded 5- or 6-membered heterocyclic group containing from 1 to 4 ring nitrogen atoms, especially N-bonded pyrrolidinyl.

"Nicotine-based hapten" as used herein refers to nicotine, either in its enantiomerically pure S or R form or a mixture thereof, which is derivatised in such manner so as to attachable to a carrier either directly, or via a cross-linker.

"Vaccine" as used herein refers to a formulation which contains the nicotine-based hapten of the present invention linked to a carrier and which is in a form that is capable of being administered to an animal. Typically, the vaccine comprises a conventional saline or buffered aqueous solution medium, for example an aluminium salt solution, in which the nicotine-based hapten-carrier conjugate is suspended or dissolved. In this form, the vaccine can be used-conveniently to prevent, ameliorate, or otherwise treat a condition. Upon introduction into a host, the vaccine is able to provoke an immune response including, but not limited to, the production of antibodies and/or cytokines and/or the activation of cytotoxic T cells, antigen presenting cells (e.g. immunoglobulins), helper T cells, dendritic cells and/or other cellular responses.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

The present invention relates to a process for preparing nicotine-based haptens that are useful components of smoking cessation vaccines.

Smoking cessation vaccines that contain nicotine-based haptens are disclosed in US 6,932,971 (Cytos), US 6,232,082 (Nabi) and US 6,656,469 (IPAB) where the haptens are linked via a chemical bridge to a phagus Qβ recombinant virus-like-particle produced in *E*. *coli*., recombinant *Psuedomonas aeruginosa* exoprotein A (rEPA) and a tetanus toxoid respectively.

Nicotine has the following chemical structure:

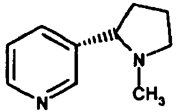

Nicotine does not provoke an immunological response in man. However it is possible to generate nicotine-specific antibodies in man when nicotine is derivatised to form a hapten that is linked to a suitable carrier.

The present invention relates to a process for preparing nicotine-based haptens of formula I: 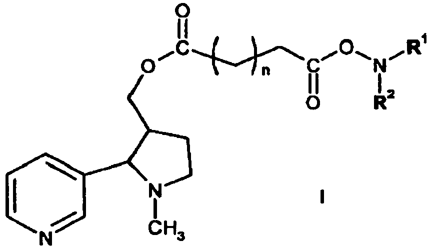 in free, salt or solvate form, wherein n is an integer from 0 to 5, and R¹ and R² together form a N-bonded 5- to 10-membered heterocyclic group containing from 1 to 4 ring nitrogen atoms and optionally containing from 1 to 4 other heteroatoms selected from the group consisting of oxygen and sulfur, said heterocyclic group being optionally substituted at 1, 2 , 3 or 4 positions by halo, cyano, hydroxy, oxo, amino, aminocarbonyl, nitro, C₁-C₄-alkyl, C₁-C₄-alkoxy or C₃-C₆-cycloalkyl.

The following suitable, preferred, more preferred or most preferred aspects of the invention may be incorporated independently, collectively or in any combination.

n is suitably an integer from 0 to 5, for example 0, 1, 2, 3, 4 or 5, but especially 1.

R¹ and R² together suitably form a N-bonded 5- to 6-membered heterocyclic group containing from 1 or 2 ring nitrogen atoms and optionally containing 1 or 2 other heteroatoms selected from the group consisting of oxygen and sulfur, said heterocyclic group being optionally substituted at 1 or 2 positions by halo, cyano, hydroxy, oxo, amino, aminocarbonyl, nitro, C₁-C₄-alkyl, C₁-C₄-alkoxy or C₃-C₆-cycloalkyl, especially oxo. For example, R¹ and R² together a pyrrolidine group substituted at positions 2 and 5 by oxo i.e. succinimidyl.

When the aforementioned N-bonded 5- to 6-membered heterocyclic group is substituted by C₁-C₄-alkyl, C₁-C₄-alkyl is suitably methyl or ethyl.

When the aforementioned N-bonded 5- to 6-membered heterocyclic group is substituted by C₁-C₄-alkoxy, C₁-C₄-alkoxy is suitably methoxy or ethoxy.

When the aforementioned N-bonded 5- to 6-membered heterocyclic group is substituted by C₃-C₈-cycloalkyl, C₃-C₈-cycloalkyl is suitably pentyl or hexyl.

The haptens include at least one asymmetric carbon atom so they exist in individual isomeric forms or as mixtures thereof, e.g. as racemic or diastereomeric mixtures. The present invention embraces preparing all individual isomers of each stereocentre (e.g. SS SR RS and RR isomers) as well as mixtures, e.g. racemic (e.g. 50:50 of two isomers or 25:25:25:25 of all four isomers); or diastereomeric mixtures, thereof.

Nicotine-based haptens of formula I are suitably nicotine-based haptens of formula la 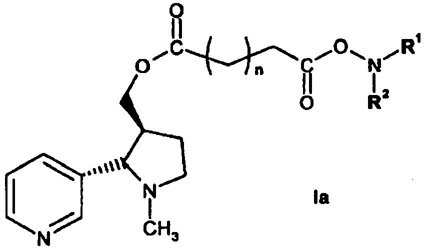 in free, salt or solvate form, which is a racemic mixture of trans enantiomers.

In an especially preferred embodiment the nicotine-based hapten of formula I is a nicotine-based hapten of formula Ib 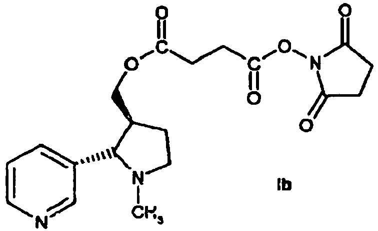 in free, salt or solvate form, which is a racemate of trans-4-nicotine methylene mono-succinate ester succinimidyl ester, also known as trans succinic acid 2,5-dioxo-pyrrolidin-1-yl ester 1-methyl-2-pyridin-3-yl-pyrrolidin-3-ylmethyl ester (C₁₉H₂₃N₃O₆).

The process for preparing the nicotine-based haptens of formula I comprises two steps, (a) and (b), which can be performed in a standard reactor (preferably with slow stirring in order not to damage the polymer structure) and filtration in a standard unit.

In step (a) a compound of formula II 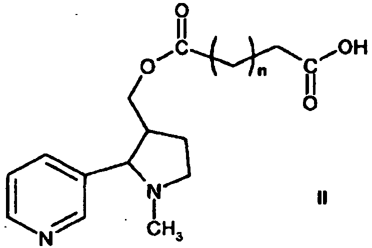 or a salt or solvate thereof,

wherein n is an integer from 0 to 5, is reacted with a compound of formula III 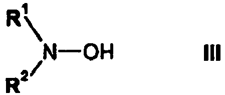 or a salt thereof,

wherein R¹ and R² are as hereinbefore defined,

and with a polymer-supported coupling agent.

In a preferred embodiment the compound of formula II is a compound of formula IIa 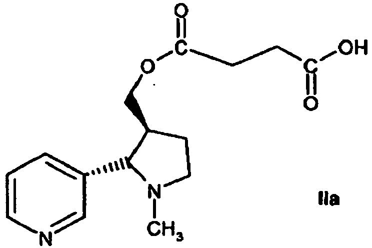 in free, salt or solvate form.

The polymer-supported coupling agent is suitably a polymer-supported solution phase synthesis reagent for producing activated acid species in the formation of amide bonds and esters.

In a preferred embodiment the polymer-supported coupling agent is a compound of formula IV 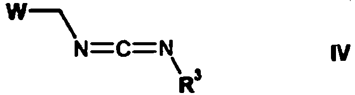 wherein W denotes a solid phase substrate chemically linked to the indicated methylene group and R³ is C₁-C₅-alkyl or C₃-C₆-cycloalkyl.

When R³ is C₁-C₅-alkyl, it is suitably ethyl or isopropyl.

When R³ is C₃-C₈-cycloalkyl, it is suitably cyclohexyl.

In an especially preferred embodiment the compound of formula IV is suitably a cyclohexyl carbodiimide resin that is commercially available from Varian Inc as StratoSpheres^{™} PL-DCC resin.

An advantage of using a polymer-supported coupling agent is that toxic by-products remain bound to the polymeric support thereby greatly simplifying the work-up procedure by avoiding the need for aqueous extractions and the removal of the solid by-product (the dicyclohexylurea). Any unreacted acid or amine species can be removed by adding an appropriate scavenger resin.

The compounds of formula II and III are preferably mixed before coming into contact with the polymer-supported coupling agent. It should be noted mixing the compound of formula II with the polymer-supported coupling agent before admixing the compound of formula III can diminish yield of nicotine-based haptens of formula I and even lead to alternative products being formed.

The reaction may be effected using known methods for reacting carboxylic acids with amino compounds and polymer-supported coupling agent (e.g. substrate-bound carbodiimide derivatives), or analogously e.g. as hereinafter described in the Examples. The reaction is conveniently carried out using an organic solvent such as 2-butanone (also known as ethyl methyl ketone or butan-2-one) i.e. the compounds of formula II and III are dissolved in the solvent in a first vessel and the polymer-supported coupling agent is swelled with the same solvent in a second vessel. The contents of the first and second vessels are combined so that the compounds of formula II and III react with an intermediate of the polymer-supported coupling agent. Suitable reaction temperatures are from 20°C to 70°C, preferably from 40°C to 60°C, but especially about from 50°C.

Increasing the temperature of the reaction would most likely increase the reaction rate, but this tends to increase the quantity and number of side-products. Higher temperatures can lead to nucleophilic ring opening of the succinimidyl group followed by a rearrangement.

In step (b) the product of step (a) is filtered to give the nicotine-based hapten of formula I in solution.

Filtering can be achieved by any art-known means.

The final product is of high purity, especially when compared to the laboratory scale process described in US 6,932,971. This is particularly important when preparing the hapten for clinically testing the vaccine. It is often difficult to purify nicotine-based haptens as this tends to lead to degradation through hydrolysis. Preferably, the purity of the nicotine hapten is greater than 80, 85 or 90% pure, as determined, for example, by ¹H NMR.

If desired the resulting solid form is dissolved in a suitable organic solvent, for example 2-butanone, for shipping and storage, which is suitably at a reduced temperature i.e. from -100° C to 20°C, for example about -80° C.

Compounds of formula II are commercially available or may be prepared by reacting a compound of formula V

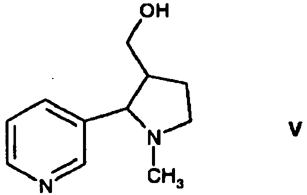

with a compound of formula VI 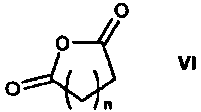 wherein n is an integer from 0 to 5. The reaction may be effected using known methods for reacting dihydro-furan-2,5-dione or analogues with alcohols, or analogously e.g. as hereinafter described in the Examples.

Compounds of formula III are either commercially available or may be obtained by known procedures for preparing hydroxylated N-heterocyclic compounds.

Compounds of formula IV are commercially available.

The compound of formula V may be obtained by reducing a compound of formula VII, 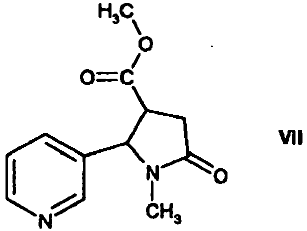 namely, 1-methyl-5-oxo-2-pyridin-3-yl-pyrrolidine-3-carboxylic acid methyl ester. The reaction may be effected using known methods for reducing esters, preferably using a reducing agent such as lithium aluminium hydride, or analogously e.g. as hereinafter described in the Examples.

Compounds of formula VI are commercially available.

The compound of formula VII may be obtained by esterifying a compound of formula VIII, 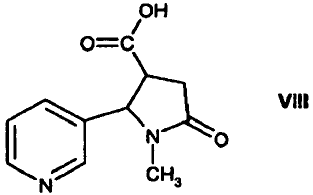 namely, 1-methyl-5-oxo-2-pyridin-3-yl-pyrrolidine-3-carboxylic acid. The reaction may be effected using known methods for esterifying carboxylic acids with alcohols to form esters, preferably using a dehydrating agent such as thionyl chloride (SOCl₂), or analogously e.g. as hereinafter described in the Examples.

In a preferred embodiment the present invention is a process for preparing a nicotine-based hapten of formula Ib 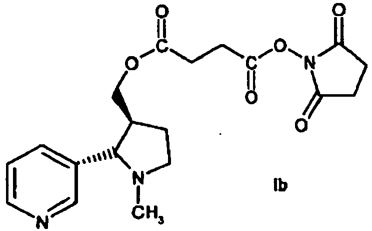 or a salt or solvate thereof, the process comprising the steps of:

(a) reacting a compound of formula IIa

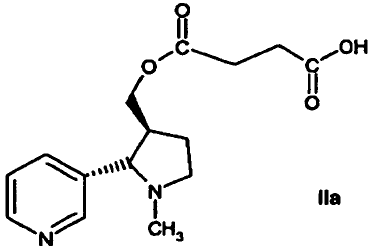

with N-hydroxy succinimide and a polymer-supported coupling agent of formula IV 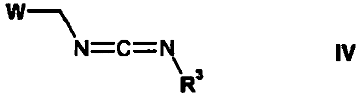 wherein W denotes a solid phase substrate chemically linked to the indicated methylene group and R³ is C₁-C₅-alkyl or C₃-C₆-cycloalkyl; and

(b) filtering the product of step (a) to give a compound of formula Ib in free, salt or solvate form.

Haptens prepared by the process of the present invention can be prepared as the free-base, or a salt or solvate form. If desired or necessary, they may be converted into various salt forms or solvates (suitably using a non-alcoholic solvent), and vice versa, in a conventional manner. Isomers, such as enantiomers and diastereomers, may be obtained in a conventional manner, e.g. by asymmetric synthesis from correspondingly asymmetrically substituted, e.g. optically active, starting materials or optically active asymmetric catalysts or optically active auxiliaries.

Haptens prepared by the process of the present invention are useful components of smoking cessation vaccines. To make such vaccines the hapten is covalently conjugated to suitable carrier, e.g. a virus-like particle (VLP), such as a VLP based on the coat proteins of an RNA phage, preferably RNA phage Qβ, as described in W004/009116. Suitable processes are described in WO04/009116, for example a process where the derivatized nicotine hapten reacts with lysine residues present on the surface of the virus-like particle coat proteins to form an amide bond. The hapten-carrier conjugate is then combined with one or more pharmaceutically acceptable formulation ingredients. Suitable formulations are, for example, described in W02007/131972, where the formulation includes at least one non-reducing saccharide, e.g. sucrose or trehalose, and at least one non-ionic surfactant, preferably to give a pH of from 5.4 to 6.6. In one embodiment, such formulations are lyophilized.

In one embodiment of the present invention, the composition further comprises an adjuvant, which preferably is aluminum containing adjuvant, preferably an aluminum salt, preferably aluminium hydroxide, preferably an aluminum containing mineral gel, most preferably alhydrogel. In one preferred embodiment of the present invention, the composition comprises from 1 mg to 2 mg, preferably from 1.2 mg to 1.7 mg, more preferably from 1.3 mg to 1.5 mg of aluminium salt, preferably aluminium hydroxide.

The vaccine is typically injected into human patients desiring an aid to smoking cessation. Suitable dosages and dosage regiments are described in WO2008/129020. For example, the dosage regiment may comprise at least a first, a second and a third administration into the human of the composition, wherein the time interval between the first administration and the second administration, and between the second administration and the third administration is at most 18 days. Preferably the time interval between the first administration and the second administration, and between the second administration and the third administration is at least three days, preferably at least four days, more preferably at least five days. In one preferred embodiment, the time interval between the first administration and the second administration, and between the second administration and the third administration is at least five days and at most 18 days.

In one preferred embodiment, during each administration a dose of at least 50 µg of the hapten-carrier conjugate, preferably at least 100 µg, or preferably at least 200 µg or at least 300 µg is administered. The dose of the hapten-carrier conjugate preferably shall not exceed 500 µg, preferably not exceeding 400 µg. In one preferred embodiment of the present invention, during each administration about 100 µg of the hapten-carrier conjugate is administered. The compositions may be administered by various methods known in the art, but will normally be administered by injection, infusion, inhalation, oral administration, or other suitable physical methods. The compositions may alternatively be administered intramuscularly, intravenously, transmucosally, transdermally or subcutaneously. In one very preferred embodiment, the administration of the composition is administered subcutaneously. Components of compositions for administration include sterile aqueous (e.g., physiological saline) or non-aqueous solutions and suspensions. Examples of non-aqueous solvents are propylene glycol, polyethylene glycol, vegetable oils such as olive oil, and injectable organic esters such as ethyl oleate

Antibodies against nicotine are induced by an immune reaction of the body to the hapten-carrier conjugate particles. It is believed that these antibodies will bind any inhaled nicotine preventing them passing through the blood-brain barrier. As such, no nicotine binding to target sites in the brain and no resulting dopamine release will occur which provides the sensation of pleasure/reward. The patient will thereby be more likely to continue to refrain from smoking.

The invention is described further by reference to the following Examples, which are illustrative only and non-limiting.

### EXAMPLES

### Preparation of starting materials

### Preparation of racemic trans-4-nicotine methylene alcohol:

Racemic trans-4-cotinine carboxylic acid (1.8 kg) is added to methanol (17 L) at room temperature, and the suspension heated to 35°C (internal temperature)(Buechi glass-lined 50 L vessel). Thionyl chloride (1.07 kg) is then added carefully within one hour at 35°C. The solution is left to stir for a further 1 hour upon which an In-Process Control (HPLC conversion) is conducted to ensure complete reaction. Toluene (12 L) is added to the reaction mixture, prior to cooling to - 5°C (internal temperature) and the solution carefully quenched with aqueous sodium hydroxide solution (2.5 kg, 10 N). Filtration to remove NaCl is undertaken before washing of the cake with methanol (6.3 L) and subsequent azeotropic removal of methanol and water by repeat distillations from toluene (total volume toluene 48 L). A second filtration (further NaCl removal) gives the racemic trans-methyl trans-4-cotinine ester as a toluene solution (concentration in the range of 9 m/m%). The solution is to be stored for a limited period due to spontaneous crystallisation. The crystals can be re-dissolved on warming to 50 °C (external temperature) for 1 hour.

The racemic trans-methyl-4-cotinine ester solution (1.91 kg as a solution in toluene) is added slowly at room temperature to a lithium aluminium hydride solution in THF (7.79 kg, 4.5 m/m%, Chemetall)(Buechi glass-lined 100 L vessel). The reaction mixture is left to stir for 4 hours upon which cellflock (1.5 kg) is added and the reaction quenched carefully with water (0.5 kg) After two hours of further stirring, the insoluble lithium and aluminium salts are removed by filtration, and the cake is washed with THF (13 L). The racemic trans-4-nicotine methylene alcohol is obtained as a solution in THF / toluene (concentration in the range of 4 m/m %).

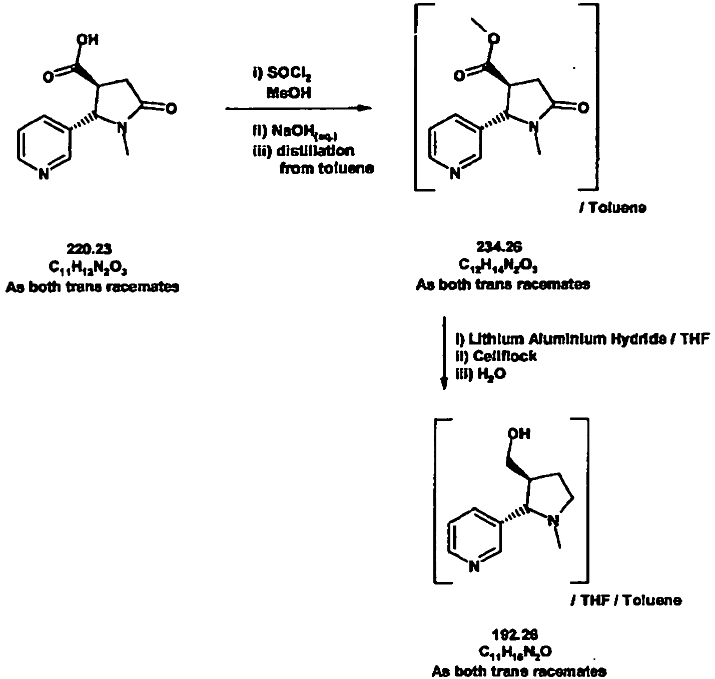

### Preparation of racemic trans-4-nicotine methylene mono-succinate ester:

Racemic trans-4-nicotine methylene alcohol as a solution in THF / toluene (1.27 kg, concentration in the range of 4 m/m %) is heated to 55 °C (internal temperature) and distillation, followed by acetonitrile addition (total volume 21.6 L) is repeated to provide the alcohol in acetonitrile (Buechi glass-lined 50 L vessel). The solution of the racemic trans-4-nicotine methylene alcohol (1.27 kg, as a solution in acetonitrile in the range of 12 m/m %) is then heated to 70 °C (internal temperature) to which a solution of succinic anhydride (VI) in acetonitrile (668 g, 23 m/m %) is slowly added. The reaction mixture is left to stir at 70 °C for 8 hours until sufficient conversion to the product (II) is confirmed by In-Process Control (HPLC conversion). A solvent switch to 2-butanone is made and the crude material chromatographed over silica (15 kg) (Filtration plate apparatus, 20 L), eluting the product with a 2-butanone/methanol mixture (1:1, 192 kg). Following analysis for purity (HPLC), selected fractions are then distilled and the solvent switched back to 100% 2-butanone.

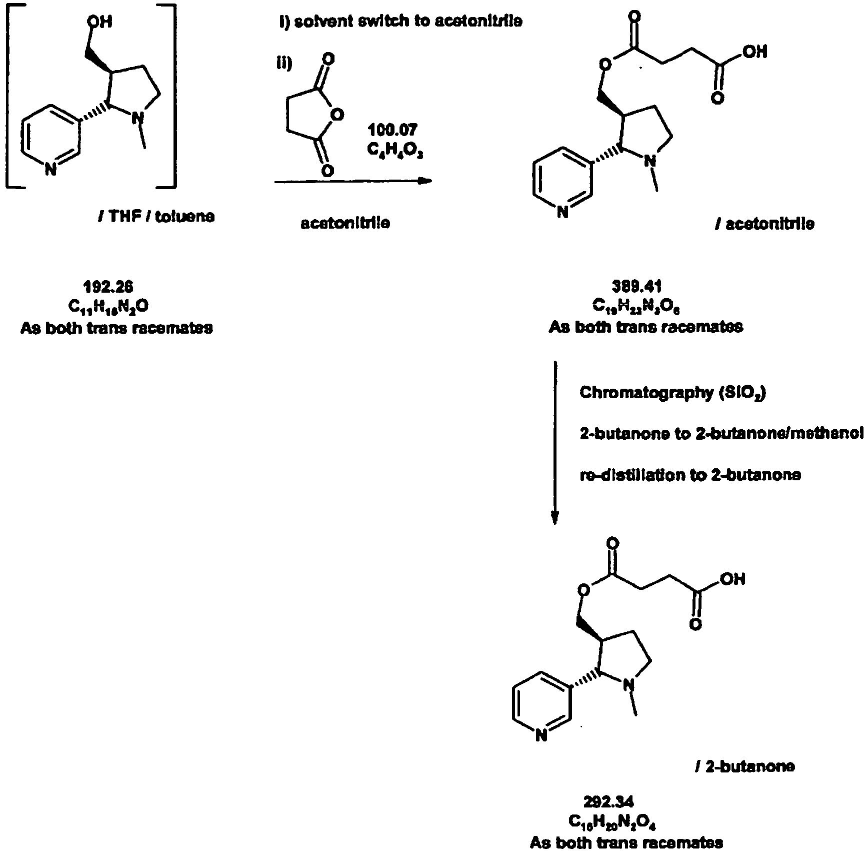

**Example 1 - Preparation of racemic trans-4-nicotine methylene mono-succinate ester succinimidyl ester**

Polymeric cyclohexylcarbodiimide resin IV (StratoSpheres^{™} PL-DCC resin ex Varian Inc) is pre-swelled in 2-butanone (8.3 L) overnight at room temperature with slow stirring (Buechi glass-lined 100 L vessel).

Trans-4-nicotine methylene mono-succinate ester (500 g in 2-butanone solution, concentration in the range of 25 m/m%) and N-hydroxy succinimide (206 g) are premixed with 2-butanone (3.5 L), stirring for 1 hour (Buechi glass-lined 50 L vessel). The mixture is slowly added to the reactor containing the swelled PL-DCC suspension which has been pre-heated to 50 °C (internal temperature). Slow stirring is continued for 2 hours.

Following successful In-Process Control (HPLC conversion), the suspension is filtered to remove the polymeric reagent and by-products, and the cake washed. The 2-butanone is then fully or partially removed by distillation using a rotary evaporator. Racemic trans-4-nicotine methylene mono-succinate ester succinimidyl ester is formed as a golden oil when fully dried with a mass of 673 g.

The final product is determined to have a purity of > 70% by HPLC assay (area comparison to an aminated analogue) and ≥ 90% by ¹H NMR.

The process is summarised in the following scheme:

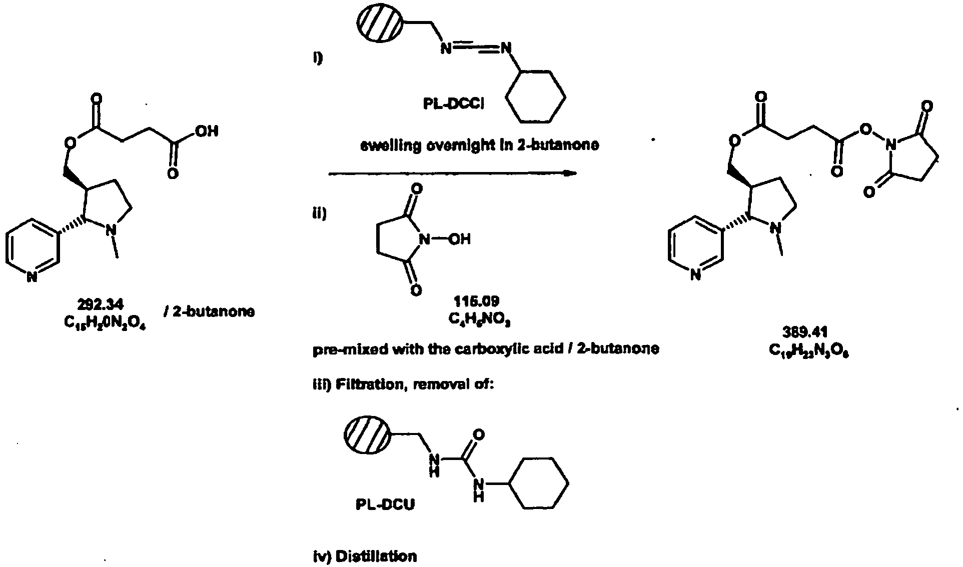

## Claims

1. A process for preparing a nicotine-based hapten of formula I 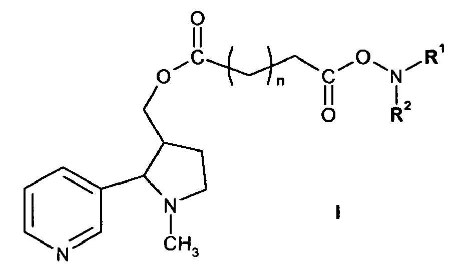 or a salt or solvate thereof,
wherein n is an integer from 0 to 5, and R¹ and R² together form a N-bonded 5- to 10-membered heterocyclic group containing from 1 to 4 ring nitrogen atoms and optionally containing from 1 to 4 other heteroatoms selected from the group consisting of oxygen and sulfur, said heterocyclic group being optionally substituted at 1, 2 , 3 or 4 positions by halo, cyano, hydroxy, oxo, amino, aminocarbonyl, nitro, C₁-C₄-alkyl, C₁-C₄-alkoxy or C₃-C₆-cycloalkyl, the process comprising the steps of:
(a) reacting a compound of formula II 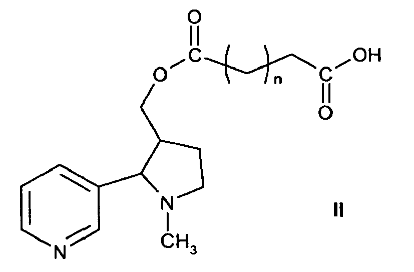 or a salt or solvate thereof,
wherein n is an integer from 0 to 5,
with a compound of formula III 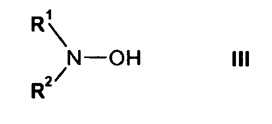 or a salt thereof,
wherein R¹ and R² together form a N-bonded 5- to 10-membered heterocyclic group containing from 1 to 4 ring nitrogen atoms and optionally containing from 1 to 4 other heteroatoms selected from the group consisting of oxygen and sulfur, said heterocyclic group being optionally substituted at 1, 2 , 3 or 4 positions by halo, cyano, hydroxy, oxo, amino, aminocarbonyl, nitro, C₁-C₄-alkyl, C₁-C₄-alkoxy or C₃-C₆-cycloalkyl,
and a polymer-supported coupling agent; and
(b) filtering the product of step (a) to give a compound of formula I in free, salt or solvate form, wherein n is an integer from 0 to 5, and R¹ and R² together form a N-bonded 5- to 10-membered heterocyclic group containing from 1 to 4 ring nitrogen atoms and optionally containing from 1 to 4 other heteroatoms selected from the group consisting of oxygen and sulfur, said heterocyclic group being optionally substituted at 1, 2 , 3 or 4 positions by halo, cyano, hydroxy, oxo, amino, aminocarbonyl, nitro, C₁-C₄-alkyl, C₁-C₄-alkoxy or C₃-C₆-cycloalkyl.

2. A process according to claim 1, wherein n of the compound of formula II is 1.

3. A process according to claim 1 or claim 2, wherein R¹ and R² of the compound of formula III together form a N-bonded 5- to 6-membered heterocyclic group containing from 1 or 2 ring nitrogen atoms and optionally containing 1 or 2 other heteroatoms selected from the group consisting of oxygen and sulfur, said heterocyclic group being optionally substituted at 1 or 2 positions by halo, cyano, hydroxy, oxo, amino, aminocarbonyl, nitro, C₁-C₄-alkyl, C₁-C₄-alkoxy or C₃-C₆-cycloalkyl.

4. A process according to claim 3, wherein R¹ and R² of the compound of formula III together form a pyrrolidine group substituted at positions 2 and 5 by oxo.

5. A process according to any one of the preceding claims, wherein the polymer-supported coupling agent is a compound of formula IV 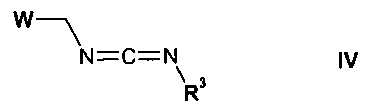 wherein W denotes a solid phase substrate chemically linked to the indicated methylene group and R³ is C₁-C₅-alkyl or C₃-C₈-cycloalkyl.

6. A process according to claim 5, wherein R³ of the compound of formula IV is ethyl, isopropyl or cyclohexyl.

7. A process according to any preceding claim, wherein step (a) is carried out at a temperature from 40°C to 60°C.

8. A process according to any one of the preceding claims, wherein step (a) is carried out in 2-butanone as solvent.

9. A process for preparing a nicotine-based hapten of formula I as defined in claim 1 that is also a compound of formula Ib 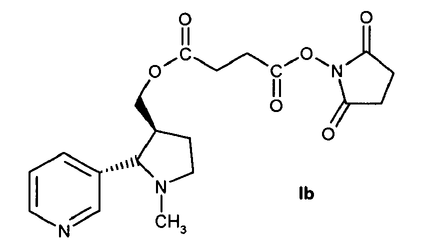 or a salt or solvate thereof, the process comprising the steps of:
(a) reacting a compound of formula IIa 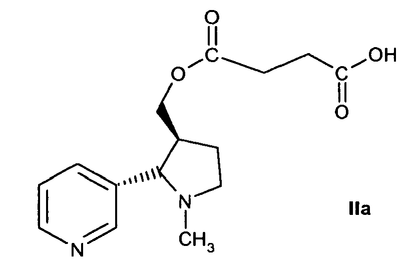 or a salt or solvate thereof, with N-hydroxy succinimide or a salt thereof, and a polymer-supported coupling agent of formula IV 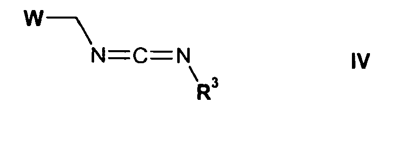 wherein W denotes a solid phase substrate chemically linked to the indicated methylene group and R³ is C₁-C₅-alkyl or C₃-C₈-cycloalkyl; and
(b) filtering the product of step (a) to give a compound of formula Ib in free, salt or solvate form.

10. A method for preparing a nicotine-based hapten-carrier conjugate, the method comprising preparing a nicotine-based hapten of formula I by the process of any one of claims 1 to 9 and covalently coupling the hapten to one or more coat proteins of a virus-like particle (VLP).

11. A method according to claim 10 wherein the VLP comprises coat proteins of an RNA phage.

## Patentansprüche

1. Verfahren zur Herstellung eines auf Nicotin basierenden Haptens der Formel I 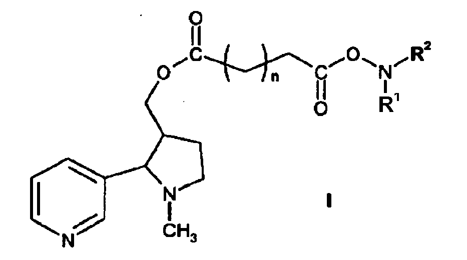 oder eines Salzes oder Solvats hiervon,
worin n für eine ganze Zahl von 0 bis 5 steht und R¹ und R² gemeinsam eine N-gebundene 5- bis 10-gliedrige heterocyclische Gruppe bilden, die 1 bis 4 Ringstickstoffatome enthält und optional 1 bis 4 weitere Heteroatome enthält, die aus der Gruppe ausgewählt sind, die aus Sauerstoff und Schwefel besteht, wobei die heterocyclische Gruppe optional an den Positionen 1, 2, 3 oder 4 mit Halogen, Cyano, Hydroxy, Oxo, Amino, Aminocarbonyl, Nitro, C₁-C₄-Alkyl, C₁-C₄-Alkoxy oder C₃-C₆-Cycloalkyl substituiert ist, wobei das Verfahren die folgenden Stufen umfasst:
(a) Umsetzen einer Verbindung der Formel II 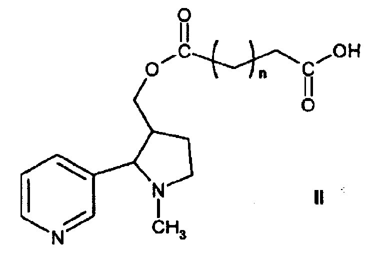 oder eines Salzes oder Solvats hiervon, worin n für eine ganze Zahl von 0 bis 5 steht, mit einer Verbindung der Formel III 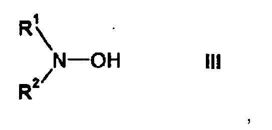 oder einem Salz hiervon, wobei R¹ und R² gemeinsam eine N-gebundene 5- bis 10-gliedrige heterocyclische Gruppe bilden, die 1 bis 4 Ringstickstoffatome enthält und optional 1 bis 4 weitere Heteroatome enthält, die aus der Gruppe ausgewählt sind, die aus Sauerstoff und Schwefel besteht, wobei die heterocyclische Gruppe optional an den Positionen 1, 2, 3 oder 4 mit Halogen, Cyano, Hydroxy, Oxo, Amino, Aminocarbonyl, Nitro, C₁-C₄-Alkyl, C₁-C₄-Alkoxy oder C₃-C₆-Cycloalkyl substituiert ist,
und einem polymergeträgerten Kupplungsmittel; und
(b) Filtern des Produkts von Stufe (a), um eine Verbindung der Formel I in freier Form, in Salz - oder Solvatform zu erhalten, wobei n für eine ganze Zahl von 0 bis 5 steht und R¹ und R² gemeinsam eine N-gebundene 5- bis 10-gliedrige heterocyclische Gruppe bilden, die 1 bis 4 Ringstickstoffatome enthält und optional 1 bis 4 weitere Heteroatome enthält, die aus der Gruppe ausgewählt sind, die aus Sauerstoff und Schwefel besteht, wobei die heterocyclische Gruppe optional an den Positionen 1, 2, 3 oder 4 mit Halogen, Cyano, Hydroxy, Oxo, Amino, Aminocarbonyl, Nitro, C₁-C₄-Alkyl, C₁-C₄-Alkoxy oder C₃-C₆-Cycloalkyl substituiert ist.

2. Verfahren nach Anspruch 1, wobei n in der Verbindung der Formel II für 1 steht.

3. Verfahren nach Anspruch 1 oder Anspruch 2, wobei R¹ und R² in der Verbindung der Formel III gemeinsam eine N-gebundene 5- bis 6-gliedrige heterocyclische Gruppe bilden, die 1 oder 2 Ringstickstoffatome enthält und optional 1 oder 2 weitere Heteroatome enthält, die aus der Gruppe ausgewählt sind, die aus Sauerstoff und Schwefel besteht, wobei die heterocyclische Gruppe optional an den Positionen 1 oder 2 mit Halogen, Cyano, Hydroxy, Oxo, Amino, Aminocarbonyl, Nitro, C₁-C₄-Alkyl, C₁-C₄-Alkoxy oder C₃-C₆-Cycloalkyl substituiert ist.

4. Verfahren nach Anspruch 3, wobei R¹ und R² in der Verbindung der Formel III gemeinsam eine Pyrrolidingruppe bilden, die an den Positionen 2 und 5 mit Oxo substituiert ist.

5. Verfahren nach einem der vorhergehenden Ansprüche, wobei das polymergeträgerte Kupplungsmittel eine Verbindung der Formel IV ist 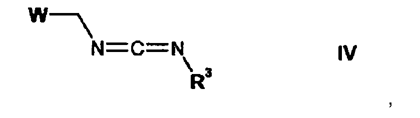 worin W ein Festphasensubstrat bezeichnet, das chemisch an die angegebene Methylengruppe gebunden ist und R³ für C₁-C₅-Alkyl oder C₃-C₈-Cycloalkyl steht.

6. Verfahren nach Anspruch 5, wobei R³ in der Verbindung der Formel IV für Ethyl, Isopropyl oder Cyclohexyl steht.

7. Verfahren nach einem der vorhergehenden Ansprüche, wobei Stufe (a) bei einer Temperatur von 40 °C bis 60 °C durchgeführt wird.

8. Verfahren nach einem der vorhergehenden Ansprüche, wobei Stufe (a) in 2-Butanon als Lösemittel durchgeführt wird.

9. Verfahren zur Herstellung eines auf Nicotin basierenden Haptens der Formel I gemäß Definition in Anspruch 1, bei dem es sich auch um eine Verbindung der Formel Ib 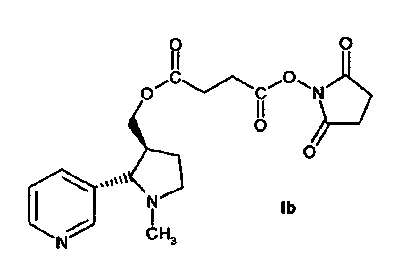 oder ein Salz oder Solvat hiervon handelt, wobei das Verfahren die folgenden Stufen umfasst:
(a) Umsetzen einer Verbindung der Formel IIa 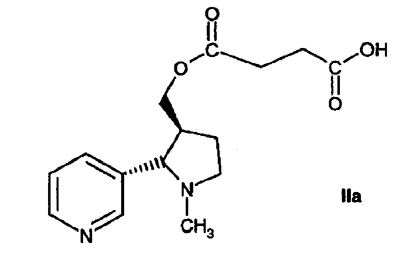 oder eines Salzes oder Solvats hiervon mit N-Hydroxysuccinimid oder einem Salz hiervon und einem polymergeträgerten Kupplungsmittel der Formel IV 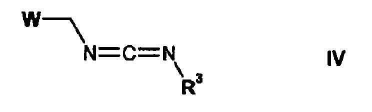 worin W ein Festphasensubstrat bezeichnet, das chemisch an die angegebene Methylengruppe gebunden ist und R³ für C₁-C₅-Alkyl oder C₃-C₈-Cycloalkyl steht; und
(b) Filtrieren des Produkts von Stufe (a), um eine Verbindung der Formel Ib in freier Form, Salz - oder Solvatform zu erhalten.

10. Verfahren zur Herstellung eines auf Nicotin basierenden Hapten-Trägerkonjugats, wobei das Verfahren das Herstellen eines auf Nicotin basierenden Haptens der Formel I mittels des Verfahrens gemäß einem der Ansprüche 1 bis 9 und ein kovalentes Kuppeln des Haptens an ein oder mehrere Hüllproteine eines virusähnlichen Teilchens (VLP) umfasst.

11. Verfahren nach Anspruch 10, wobei das VLP Hüllproteine eines RNA-Phagen umfasst.

## Revendications

1. Procédé de préparation d'un haptène à base de nicotine de formule I 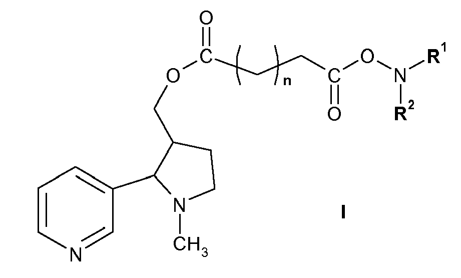 ou d'un sel ou solvate de celui-ci,
dans laquelle n est un nombre entier de 0 à 5, et R¹ et R² forment ensemble un groupe hétérocyclique à 5 à 10 chaînons et à liaison N, contenant de 1 à 4 atomes d'azote de cycle et contenant éventuellement de 1 à 4 autres hétéroatomes choisis dans le groupe constitué par l'oxygène et le soufre, ledit groupe hétérocyclique étant éventuellement substitué en position 1, 2, 3 ou 4 par un substituant halogéno, cyano, hydroxy, oxo, amino, aminocarbonyle, nitro, alkyle en C₁-C₄, alcoxy en C₁-C₄ ou cycloalkyle en C₃-C₆, le procédé comprenant les étapes consistant à:
(a) faire réagir un composé de formule II 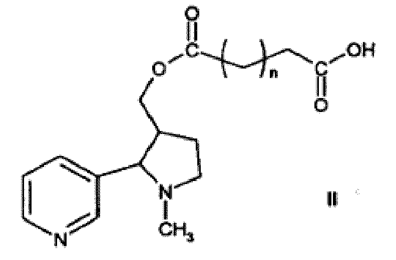 ou un sel ou solvate de celui-ci,
dans laquelle n est un nombre entier de 0 à 5, avec un composé de formule III 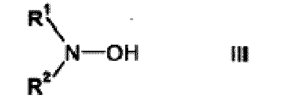 ou un sel de celui-ci, dans laquelle R¹ et R² forment ensemble un groupe hétérocyclique à 5 à 10 chaînons et à liaison N, contenant de 1 à 4 atomes d'azote de cycle et contenant éventuellement de 1 à 4 autres hétéroatomes choisis dans le groupe constitué par l'oxygène et le soufre, ledit groupe hétérocyclique étant éventuellement substitué en position 1, 2, 3 ou 4 par un substituant halogéno, cyano, hydroxy, oxo, amino, aminocarbonyle, nitro, alkyle en C₁-C₄, alcoxy en C₁-C₄ ou cycloalkyle en C₃-C₆,
et un agent de couplage sur support polymère; et
(b) filtrer le produit de l'étape (a) pour donner un composé de formule I sous forme libre, de sel ou de solvate, dans laquelle n est un nombre entier de 0 à 5, et R¹ et R² forment ensemble un groupe hétérocyclique à 5 à 10 chaînons et à liaison N, contenant de 1 à 4 atomes d'azote de cycle et contenant éventuellement de 1 à 4 autres hétéroatomes choisis dans le groupe constitué par l'oxygène et le soufre, ledit groupe hétérocyclique étant éventuellement substitué en position 1, 2, 3 ou 4 par un substituant halogéno, cyano, hydroxy, oxo, amino, aminocarbonyle, nitro, alkyle en C₁-C₄, alcoxy en C₁-C₄ ou cycloalkyle en C₃-C₆.

2. Procédé selon la revendication 1, dans lequel le n du composé de formule I est égal à 1.

3. Procédé selon la revendication 1 ou la revendication 2, dans lequel les R¹ et R² du composé de formule III forment ensemble un groupe hétérocyclique à 5 à 6 chaînons et à liaison N, contenant 1 ou 2 atomes d'azote de cycle et contenant éventuellement 1 ou 2 autres hétéroatomes choisis dans le groupe constitué par l'oxygène et le soufre, ledit groupe hétérocyclique étant éventuellement substitué en position 1 ou 2 par un substituant halogéno, cyano, hydroxy, oxo, amino, aminocarbonyle, nitro, alkyle en C₁-C₄, alcoxy en C₁-C₄ ou cycloalkyle en C₃-C₆.

4. Procédé selon la revendication 3, dans lequel les R¹ et R² du composé de formule III forment ensemble un groupe pyrrolidine substitué en positions 2 et 5 par un oxo.

5. Procédé selon l'une quelconque des revendications précédentes, dans lequel l'agent de couplage sur support polymère est un composé de formule IV 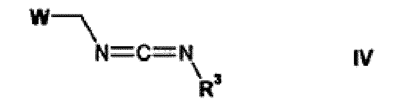 dans laquelle W désigne un substrat en phase solide lié chimiquement au groupe méthylène indiqué et R³ est un groupe alkyle en C₁-C₅ ou cycloalkyle en C₃-C₈.

6. Procédé selon la revendication 5, dans lequel le groupe R³ du composé de formule IV est un groupe éthyle, isopropyle ou cyclohexyle.

7. Procédé selon l'une quelconque des revendications précédentes, dans lequel l'étape (a) est réalisée à une température de 40°C à 60°C.

8. Procédé selon l'une quelconque des revendications précédentes, dans lequel l'étape (a) est réalisée dans de la 2-butanone en tant que solvant.

9. Procédé de préparation d'un haptène à base de nicotine de formule I telle que définie dans la revendication 1, qui est également un composé de formule Ib 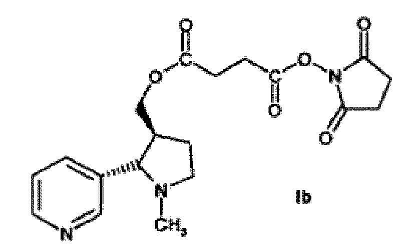 ou un sel ou solvate de celui-ci, le procédé comprenant les étapes consistant à:
(a) faire réagir un composé de formule IIa 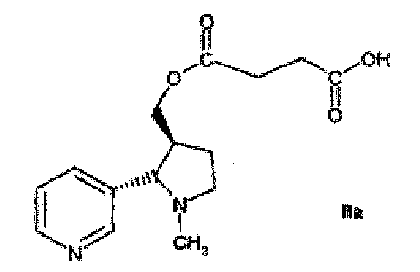 ou un sel ou solvate de celui-ci, avec du N-hydroxysuccinimide ou un sel de celui-ci,
et un agent de couplage sur support polymère de formule IV 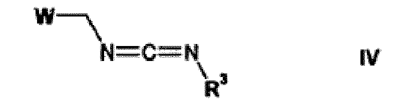 dans laquelle W désigne un substrat en phase solide lié chimiquement au groupe méthylène indiqué et R³ est un groupe alkyle en C₁-C₅ ou cycloalkyle en C₃-C₈, et
(b) filtrer le produit de l'étape (b) pour donner un composé de formule Ib sous forme libre, de sel ou de solvate.

10. Procédé de préparation d'un conjugué haptène à base de nicotine-vecteur, le procédé comprenant la préparation d'un haptène à base de nicotine de formule I par le procédé de l'une quelconque des revendications 1 à 9 et le couplage covalent de l'haptène à une ou plusieurs protéines d'enveloppe d'une pseudo-particule virale (virus-like particle, VLP).

11. Procédé selon la revendication 10, dans lequel la VLP comprend les protéines d'enveloppe d'un phage à ARN.
